# EUROPÄISCHE PATENTSCHRIFT

(11) **EP 0 603 650 B1**
(45) Veröffentlichungstag und Bekanntmachung des Hinweises auf die Patenterteilung: **02.04.1997**
(21) Anmeldenummer: 93119784.2
(22) Anmeldetag: 08.12.1993
(51) Int. Cl.: C07C 279/22, A61K 31/155

(54) **3,5-Substituierte Aminobenzoylguanidine, Verfahren zu ihrer Herstellung, ihre Verwendung als Medikament oder Diagnostikum sowie sie enthaltendes Medikament**
3,5-Substituted amino benzoylguanidines, process for their preparation, their use as medicine or diagnostic agent, as well as a medicine containing them
Aminobenzoylguanidines 3,5, substituées, procédé de leur préparation, leur application à titre de médicament ou d'agent diagnostique ainisi que un médicament les contenant

(30) Priorität: 16.12.1992 DE 4242554
(43) Veröffentlichungstag der Anmeldung: 29.06.1994
(73) Patentinhaber: HOECHST AKTIENGESELLSCHAFT, 65929 Frankfurt am Main (DE)
(72) Erfinder: Lang, Hans-Jochen, Dr., D-65719 Hofheim/Taunus (DE); Englert, Heinrich, Dr., D-65719 Hofheim/Taunus (DE); Weichert, Andreas, Dr., D-60529 Frankfurt/Main (DE); Kleemann, Heinz-Werner, Dr., D-61350 Bad Homburg (DE); Scholz, Wolfgang, Dr., D-65760 Eschborn (DE); Albus, Udo, Dr., D-61197 Florstadt (DE)

(56) Entgegenhaltungen:
- EP-A- 0 416 499
- US-A- 3 780 027
- CHEMICAL ABSTRACTS, vol. 81, no. 23, 9. Dezember 1974, Columbus, Ohio, US; abstract no. 147547q, P. MILDNER ET AL. 'Inhibition of urease by some triazole, urea,and guanidine derivatives' Seite 168 ;

## Beschreibung

Die Erfindung betrifft Benzoylguanidine der Formel I 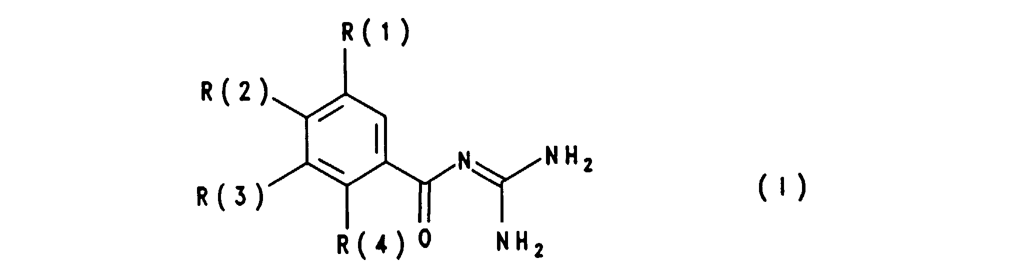 worin bedeuten:
einer der Substituenten R(1), R(2), R(3) oder R(4):
eine Aminogruppe 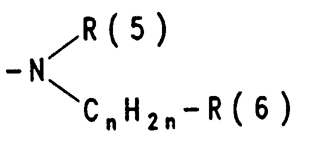 mit
- R(5) =: Wasserstoff oder C₍₁₋₆₎-Alkyl,
- n =: Null, 1, 2, 3 oder 4,
- R(6) =: H, C₍₁₋₄₎-Alkyl,
worin eine CH₂-Gruppe durch 1 S-Atom oder eine Gruppe NR(7) mit R(7) = Wasserstoff, Methyl oder Ethyl ersetzt sein kann,
C₍₃₋₈₎-Cycloalkyl, Phenyl,
das unsubstituiert ist oder 1, 2 oder 3 Substituenten aus der Reihe F, Cl, Br, Methyl, Methoxy, -NR(8)R(9) trägt mit R(8) und R(9) gleich H, Methyl, Ethyl,
und worin
R(5) und R(6) auch gemeinsam mit dem Stickstoffatom einen 5-, 6- oder 7-gliedrigen Ring bilden können, in welchem 1 C-Atom durch Sauerstoff, S oder NR(10) im Sinne von Morpholino, Thiomorpholino und Piperazino mit R(10) gleich H, C₍₁₋₃₎-Alkyl oder Benzyl ersetzt sein kann,
und die jeweils anderen Substituenten R(1), R(2), R(3), R(4):
Wasserstoff, F, Cl, Br, I, CN, CF₃, NO₂, CF₃-O-, CₘF₂ₘ₊₁-CH₂-O-
mit m = 1, 2 oder 3,

R(11)-C_{q}H_{2q}-Xₚ-

mit
- q =: Null, 1, 2, 3 oder 4,
- p =: Null oder 1,
- X =: Sauerstoff oder NR(12) bedeuten,
R(12) = H oder C₍₁₋₃₎-Alkyl,
- R(11) =: Wasserstoff, C₍₁₋₆₎-Alkyl, C₍₃₋₈₎-Cycloalkyl, Phenyl,
das unsubstituiert oder substituiert ist mit 1, 2 oder 3 Substituenten aus der Gruppe F, Cl, CH₃, CH₃-O-, NR(13)R(14) mit R(13), R(14) gleich H, Methyl oder Ethyl
und wobei R(1) und R(4) nicht gleichzeitig Wasserstoff ist.

Bevorzugt sind Verbindungen der Formel 1, worin bedeuten:
einer der Substituenten R(1), R(2), R(3) oder R(4):
eine Aminogruppe 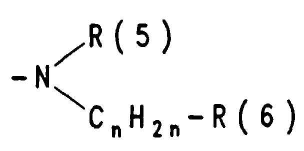 mit
- R(5) =: Wasserstoff oder C₍₁₋₄₎-Alkyl,
n = Null, 1 oder 2, und
- R(6) =: H, C₍₁₋₄₎-Alkyl,
worin eine CH₂-Gruppe durch ein S-Atom oder eine Gruppe NR(7) mit R(7) = H, Methyl oder Ethyl ersetzt sein kann,
C₍₃₋₆₎-Cycloalkyl, Phenyl,
das unsubstituiert ist oder 1, 2 oder 3 Substituenten aus der Gruppe F, Cl, Br, Methyl, Methoxy, -NR(8)R(9) trägt mit R(8) und R(9) gleich H, Methyl, Ethyl,
und worin R(5) und R(6) auch gemeinsam mit dem Stickstoffatom einen 5-, 6- oder 7-gliedrigen Ring bilden kann,
und die jeweils anderen Substituenten R(1), R(2), R(3), R(4):
Wasserstoff, F, Cl, Br, l, CF₃, NO₂, CₘF₂ₘ₊₁-CH₂-O-
mit m = 1, 2 oder 3,

R(11)-C_{q}H_{2q}-Xₚ-

mit
- q =: Null, 1 oder 2,
- p =: Null oder 1,
- X =: Sauerstoff,
- R(11) =: Wasserstoff, C₍₁₋₆₎-Alkyl, C₍₅₋₆₎-Cycloalkyl, Phenyl,
das unsubstituiert ist oder substituiert ist mit 1 oder 2 Substituenten aus der Gruppe F, Cl, Methyl und CH₃-O-,
und wobei R(1) und R(4) nicht gleichzeitig Wasserstoff ist.

Besonders bevorzugt sind Verbindungen der Formel I, worin bedeuten:
R(4) Wasserstoff,
einer der Substituenten R(1), R(2) oder R(3):
eine Aminogruppe 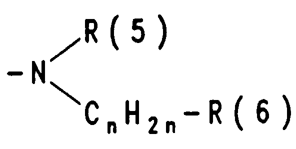 mit
- R(5) =: Wasserstoff oder C₍₁₋₄₎-Alkyl,
n = Null, 1 oder 2, und
- R(6) =: H, C₍₁₋₄₎-Alkyl, C₍₅₋₆₎-Cycloalkyl, Phenyl,
das unsubstituiert ist oder 1 oder 2 Substituenten aus der Gruppe F, Cl und Methyl trägt
und die jeweils anderen Substituenten R(1), R(2), R(3) bedeuten:
Wasserstoff, F, Cl, CF₃, R(11)-C_{q}-H_{2q}-Xₚ- mit
- q =: Null bis 2,
- p =: Null oder 1,
- X =: Sauerstoff,
- R(11) =: Wasserstoff, C₍₁₋₄₎-Alkyl, C₍₅₋₆₎-Cycloalkyl, Phenyl,
das unsubstituiert ist oder substituiert mit 1 oder 2 Substituenten aus der Gruppe F, Cl, Methyl und CH₃-O-,
und wobei R(1) und R(4) nicht Wasserstoff bedeuten.

Ganz besonders bevorzugt sind die Verbindungen 2-Amino-3,5-dichlorbenzoyl-guanidin-hydrochlorid, 5-Trifluormethyl-3-N,N-dimethylaminobenzoyl-guanidin-hydrochlorid, 4-Amino-3,5-dibrombenzoyl-guanidin-hydrochlorid sowie deren pharmakologisch verträgliche Salze.

Enthält einer der Substituenten R(1) bis R(4) ein Asymmetriezentrum, so gehören sowohl S als auch R konfigurierte Verbindungen zur Erfindung. Die Verbindungen können als optische Isomere, als Diastereomere, als Racemate oder als Gemische derselben vorliegen.

Die bezeichneten Alkylreste können sowohl geradkettig als auch verzweigt vorliegen.

Die Erfindung betrifft weiterhin ein Verfahren zur Herstellung einer Verbindung I, dadurch gekennzeichnet, daß man eine Verbindung der Formel II 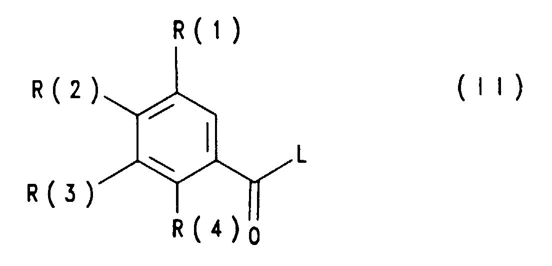 mit Guanidin umsetzt, worin R(1) bis R(4) die angegebene Bedeutung besitzen und L für eine leicht nucleophil substituierbare leaving group steht.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Benzoesäure-derivaten (Formel II, L = OH) herstellen, wie beispielsweise die Methylester der Formel II mit L = OCH₃ durch Behandeln mit gasförmigem HCl in Methanol, die Imidazolide der Formel II durch Behandeln mit Carbonyldiimidazol (L = 1-Imidazolyl, Staab, Angew. Chem. Int. Ed. Engl. 1, 351-367 (1962)), die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)methylen)amino]-1,1,3,3-tetramethyluronium-tetrafluoborat ("TOTU")(Proceedings of the 21. European Peptide Symposium, Peptides 1990, Editors E. Giralt and D. Andreu, Escom, Leiden, 1991). Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel I mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol oder THF zwischen 20°C und Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II und III verwendet werden.

Wenn L = Cl bedeutet, arbeitet man vorteilhaft unter Zusatz eines Säurefängers, z.B. in Form von überschüssigem Guanidin zur Abbindung der Halogenwasserstoffsäure.

Ein Teil der zugrundeliegenden Benzoesäurederivate der Formel II sind bekannt und in der Literatur beschrieben. Die unbekannten Verbindungen der Formel II können nach literaturbekannten Methoden hergestellt werden.

Carbonsäuren oder deren Ester der Formel II (z.B. L = -OH oder -O-Methyl) mit R(2) in der Bedeutung von Halogen oder R(3) in der Bedeutung von Nitro können als vielseitige Ausgangsverbindungen für weitere Carbonsäuren der Formel II dienen, wobei das Halogen in der Position R(2) sehr bequem in bekannter Weise gegen zahlreiche nucleophile Reagenzien, wie Mercaptane R(10)-SH, Phenole R(10)-OH oder primäre oder sekundäre Amine 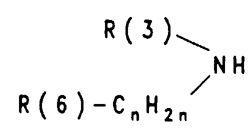 unter Bildung weiterer Benzoesäurederivate II mit L = -OH bzw. -O-Methyl ausgetauscht werden kann oder Nitro nach reduktiver Umwandlung in NH₂ in zahlreichen Reaktionen (Alkylierungen, Acylierungen, Diazotierungen mit nachfolgenden Sandmeier-, Ullmann- Meerwein- usw.) in weitere Benzoesäurederivate II mit L = -OH bzw. -O-Methyl übergeführt werden können.

Benzoylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.
Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 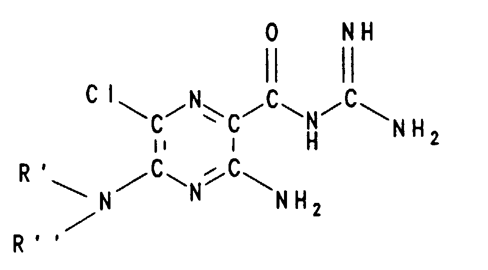 Amilorid: R',R" = H
Dimethylamilorid: R',R" = CH₃
Ethylisopropylamilorid: R' = C₂H₅, R'' = CH(CH₃)₂

Darüberhinaus sind Untersuchungen bekannt geworden, die auf antiarrhythmische Eigenschaften von Amilorid hinweisen (Circulation 79, 1257-63 (1989). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten (Eur. Heart J. 9 (suppl.1): 167 (1988) (book of abstracts). So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

In der US-Patentschrift 5 091 394 sind Benzoylguanidine beschrieben, die in der dem Rest R(1) und R(4) entsprechenden Stellung ein Wasserstoffatom tragen.

In der Literaturstelle Kumamoto Pharm. Bull. [1966], S.7-13 werden weitere Benzoylguanidine aufgeführt.

Im US-Patent 3 780 027 werden Acylguanidine beansprucht, die strukturell den Verbindungen der Formel I ähnlich sind und sich von im Handel befindlichen Schleifendiuretika, wie Bumetanid, ableiten. Entsprechend wird für diese Verbindungen eine starke salidiuretische Wirksamkeit berichtet.

Es war daher überraschend, daß die erfindungsgemäßen Verbindungen keine unerwünschten und nachteiligen salidiuretischen, jedoch sehr gute antiarrhythmische Eigenschaften aufweisen, wie sie beispielsweise bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindung auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden. Außerdem zeigen die Verbindungen I gastroprotektive Eigenschaften infolge Hemmung der Magensäuresekretion.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindung der Formel I für die präventive Therapie zur Verhinderung der Genese des Bluthochdrucks, beispielweise der essentiellen Hypertonie, geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z.B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage: Wasser, physiologische Kochsalzlösung oder Alkohole, z.B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z.B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.
Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise 0,01 mg/kg bis 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z.B. bis zu 4 Einzeldosen pro Tag. Insbesondere bei i.v. Anwendung, etwa bei einem Infarktpatienten auf der Intensivstation können bis zu 200 mg pro Tag notwendig werden.

### Experimenteller Teil

### Allgemeine Vorschrift zur Herstellung von Benzoyl-guanidinen (I) aus Benzoesäuren (II, L = OH)

0,01 M des Benzoesäurederivates der Formel II löst bzw. suspendiert man in 60 ml wasserfreiem Tetrahydrofuran (THF) und versetzt sodann mit 1,78 g (0,011 M) Carbonyldiimidazol. Nach dem Rühren über 2 Stunden bei Zimmertemperatur werden 2,95 g (0,05 M) Guanidin in die Reaktionslösung eingetragen. Nach dem Rühren über Nacht destilliert man das THF unter vermindertem Druck (Rotavapor) ab, versetzt mit Wasser, stellt mit 2N HCL auf pH 6-7 und filtriert das entsprechende Benzoylguanidin (Formel I) ab. Die so erhaltenen Benzoylguanidine können durch Behandeln mit wäßriger oder methanolischer Salzsäure oder anderen pharmakologisch verträglichen Säuren in die entsprechenden Salze übergeführt werden.

### Beispiel 1:

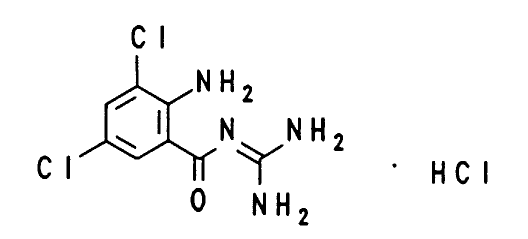

2-Amino-3,5-dichlorbenzoyl-guanidin-hydrochlorid
erhält man nach der allgemeinen Vorschrift aus 2-Amino-3,5-dichlorbenzoesäure;
farblose kristalline Substanz; Schmp. 190°C (Zers.).

### Beispiel 2:

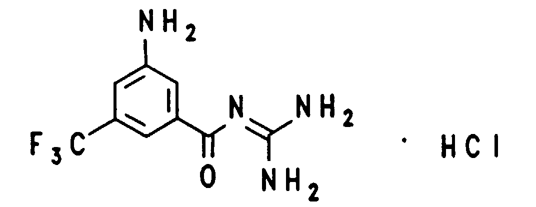

3-Amino-5-trifluormethylbenzoyl-guanidin-hydrochlorid
erhält man nach der allgemeinen Vorschrift aus 3-Amino-5-trifluormethylbenzoesäure. Farblose kristalline Substanz; Schmp. 225°C.

### Beispiel 3:

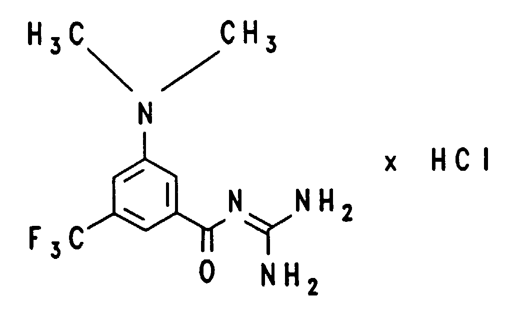

5-Trifluormethyl-3-N,N-dimethylaminobenzoyl-guanidin-hydrochlorid erhält man nach der allgemeinen Vorschrift aus 5-Trifluormethyl-3-N,N-dimethylaminobenzoesäure; farblose kristalline Substanz; Schmp. 277°C.

5-Trifluormethyl-3-N,N-dimethylaminobenzoesäure (Schmp. 194-197°C) wurde durch Umsetzung von 3-Amino-5-trifluormethylbenzoesäure mit der 3-fachen äquivalenten Menge Methyliodid in DMF in Gegenwart von fein gemahlenen wasserfreiem Kaliumcarbonat bei 60°C über mehrere Stunden und anschließende Hydrolyse des so erhaltenen 5-Trifluormethyl-3-N,N-dimethylaminobenzoesäuremethylesters (Schmp. 55-60°C) mit wäßrig-methanolischer Natronlauge und anschließendes Sauer-Stellen mit 2N HCl auf pH 1-2 erhalten.

### Beispiel 4:

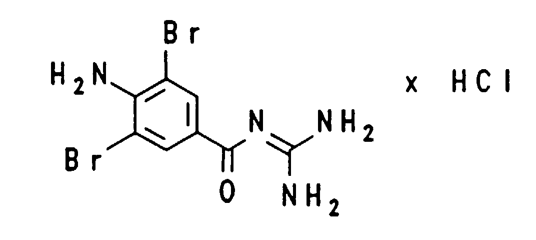

4-Amino-3,5-dibrombenzoyl-guanidin-hydrochlorid
erhält man nach der allgemeinen Vorschrift aus 4-Amino-3,5-dibrombenzoesäure. Kristalline Substanz; Schmp. 266-269°C.

### Beispiel 5:

4-Amino-3,5-dimethylbenzoyl-guanidin-dihydrochlorid
erhält man nach der allgemeinen Vorschrift aus 4-Amino-3,5-dimethylbenzoesäure. Kristalline Substanz; Schmp. > 330°C.

### Beispiel 6:

3-Brom-5-methylbenzoyl-guanidin-hydrochlorid
erhält man nach der allgemeinen Vorschrift aus 3-Brom-5-methylbenzoesäure. Kristalline Substanz; Schmp. > 300°C.

### Beispiel 7:

3,5-Dibrom-4-(1-butylamino)benzoyl-guanidin-dihydrochlorid
erhält man nach der allgemeinen Vorschrift aus 3,5-Dibrom-4-(1-butylamino)benzoesäure. Kristalline Substanz; Schmp. 214-215°C.

### Beispiel 8:

3-Amino-5-chlorbenzoyl-guanidin-dihydrochlorid
erhält man nach der allgemeinen Vorschrift aus 3-Amino-5-chlorbenzoesäure. Kristalline Substanz; Schmp. 270°C.

### Beispiel 9:

3-N,N-Dimethylamino-5-chlorbenzoyl-guanidin-dihydrochlorid
erhält man nach der allgemeinen Vorschrift aus 3-N,N-Dimethylamino-5-chlorbenzoesäure. Kristalline Substanz; Schmp. 217°C.

### Beispiel 10:

3,5-Dimethyl-4-N,N-dimethylamino-5-chlorbenzoyl-guanidin-dihydrochlorid
erhält man nach der allgemeinen Vorschrift aus 3,5-Dimethyl-4-N,N-dimethylamino-5-chlorbenzoesäure. Kristalline Substanz; Schmp. 203°C.

### Beispiel 11:

2-Amino-4,5-dimethoxybenzoyl-guanidin-dihydrochlorid
erhält man nach der allgemeinen Vorschrift aus 2-Amino-4,5-dimethoxybenzoesäure. Kristalline Substanz; Schmp. 177°C.

### Beispiel 12:

2-Amino-4,5-dibrombenzoyl-guanidin-dihydrochlorid
erhält man nach der allgemeinen Vorschrift aus 2-Amino-4,5-dibrombenzoesäure. Kristalline Substanz; Schmp. > 310°C.

### Beispiel 13:

5-Chlor-3-(4-chlorbenzylamino)benzoyl-guanidin-dihydrochlorid
erhält man nach der allgemeinen Vorschrift aus 5-Chlor-3-(4-chlorbenzylamino)benzoesäure. Kristalline Substanz; Schmp. 168°C.

### Beispiel 14:

4-Amino-3-chlor-5-methylbenzoyl-guanidin-dihydrochlorid
erhält man nach der allgemeinen Vorschrift aus 4-Amino-3-chlor-5-methylbenzoesäure. Kristalline Substanz; Schmp. 232-234°C.

### Beispiel 15:

4-Amino-3,5-dichlorbenzoyl-guanidin-dihydrochlorid
erhält man nach der allgemeinen Vorschrift aus 4-Amino-3,5-dichlorbenzoesäure. Kristalline Substanz; Schmp. 273°C.

### Beispiel 16:

4-Amino-3,5-dichlorbenzoyl-guanidin-dihydrochlorid
erhält man nach der allgemeinen Vorschrift aus 4-Amino-3,5-dichlorbenzoesäure. Kristalline Substanz; Schmp. 273°C.

### Beispiel 17:

3-Chlor-5-ethylaminobenzoyl-guanidin-dihydrochlorid
erhält man nach der allgemeinen Vorschrift aus 3-Chlor-5-ethylaminobenzoesäure. Kristalline Substanz; Schmp. 235°C.

### Beispiel 18:

5-Chlor-2-dimethylaminobenzoyl-guanidin-hydrochlorid
erhält man nach der allgemeinen Vorschrift aus 5-Chlor-2-dimethylaminobenzoesäure. Kristalline Substanz; Schmp. 135°C.

### Beispiel 19:

3-Brom-4-methyl-5-dimethylaminobenzoyl-guanidin-hydrochlorid
erhält man nach der allgemeinen Vorschrift aus 3-Brom-4-methyl-5-dimethylaminobenzoesäure. Kristalline Substanz; Schmp. 185°C.

### Beispiel 20:

3-Methylamino-5-trifluormethylbenzoyl-guanidin-dihydrochlorid
erhält man nach der allgemeinen Vorschrift aus 3-Methylamino-5-trifluormethylbenzoesäure. Kristalline Substanz; Schmp. 163°C.

### Beispiel 21:

3-Chlor-5-methyl-4-dimethylaminobenzoyl-guanidin-dihydrochlorid
erhält man nach der allgemeinen Vorschrift aus 3-Chlor-5-methyl-4-dimethylaminobenzoesäure. Kristalline Substanz; Schmp. 171°C.

### Beispiel 22:

3,5-Dichlor-4-dimethylaminobenzoyl-guanidin-hydrochlorid
erhält man nach der allgemeinen Vorschrift aus 3,5-Dichlor-4-dimethylaminobenzoesäure. Kristalline Substanz; Schmp. 183°C.

### Beispiel 23:

2-Amino-3-methylbenzoyl-guanidin-hydrochlorid
erhält man nach der allgemeinen Vorschrift aus 2-Amino-3-methylbenzoesäure. Kristalline Substanz; Schmp. > 270°C.

### Beispiel 24:

3-Chlor-4-methoxy-5-dimethylaminobenzoyl-guanidin-dihydrochlorid
erhält man nach der allgemeinen Vorschrift aus 3-Chlor-4-methoxy-5-dimethylaminobenzoesäure. Kristalline Substanz; Schmp. 148°C.

### Beispiel 25:

3,5-Dibrom-4-(4-fluorbenzylamino)benzoyl-guanidin-hydrochlorid
erhält man nach der allgemeinen Vorschrift aus 3,5-Dibrom-4-(4-fluorbenzylamino)benzoesäure. Kristalline Substanz; Schmp. 206°C.

## Patentansprüche

1. Benzoylguanidine der Formel I 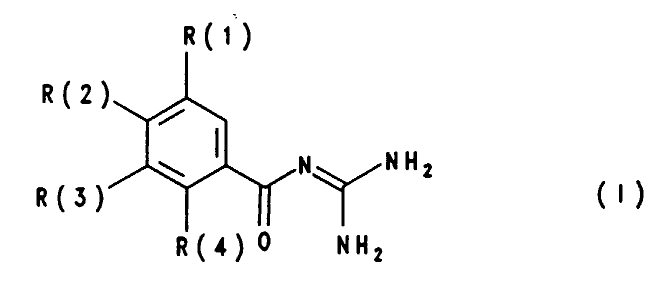 worin bedeuten:
einer der Substituenten R(1), R(2), R(3) oder R(4):
eine Aminogruppe 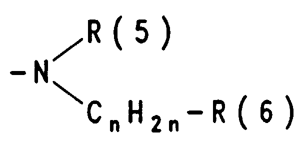 mit
R(5) = Wasserstoff oder C₍₁₋₆₎-Alkyl,
n = Null, 1, 2, 3 oder 4,
R(6) = H, C₍₁₋₄₎-Alkyl,
worin eine CH₂-Gruppe durch 1 S-Atom oder eine Gruppe NR(7) mit R(7) = Wasserstoff, Methyl oder Ethyl ersetzt sein kann,
C₍₃₋₈₎-Cycloalkyl, Phenyl,
das unsubstituiert ist oder 1, 2 oder 3 Substituenten aus der Reihe F, Cl, Br, Methyl, Methoxy, -NR(8)R(9) trägt mit R(8) und R(9) gleich H, Methyl, Ethyl,
und worin
R(5) und R(6) auch gemeinsam mit dem Stickstoffatom einen 5-, 6- oder 7-gliedrigen Ring bilden können, in welchem 1 C-Atom durch Sauerstoff, S oder NR(10) mit R(10) gleich H, C₍₁₋₃₎-Alkyl oder Benzyl ersetzt sein kann,
und die jeweils anderen Substituenten R(1), R(2), R(3), R(4):
Wasserstoff, F, Cl, Br, I, CN, CF₃, NO₂, CF₃-O-, CₘF₂ₘ₊₁-CH₂-O-
mit m = 1, 2 oder 3,
R(11)-C_{q}H_{2q}-Xₚ-
mit
q = Null, 1, 2, 3 oder 4,
p = Null oder 1,
X = Sauerstoff oder NR(12) bedeuten,
R(12) = H oder C₍₁₋₃₎-Alkyl,
R(11) = Wasserstoff, C₍₁₋₆₎-Alkyl, C₍₃₋₈₎-Cycloalkyl, Phenyl,
das unsubstituiert oder substituiert ist mit 1, 2 oder 3 Substituenten aus der Gruppe F, Cl, CH₃, CH₃-O-, NR(13)R(14) mit R(13), R(14) gleich H, Methyl oder Ethyl
und wobei R(1) und R(4) nicht gleichzeitig Wasserstoff ist.

2. Verbindung der Formel I nach Anspruch 1, worin bedeuten:
einer der Substituenten R(1), R(2), R(3) oder R(4):
eine Aminogruppe 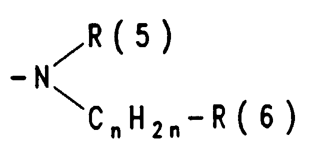 mit
R(5) = Wasserstoff oder C₍₁₋₄₎-Alkyl,
n = Null, 1 oder 2, und
R(6) = H, C₍₁₋₄₎-Alkyl,
worin eine CH₂-Gruppe durch ein S-Atom oder eine Gruppe NR(7) mit R(7) = H, Methyl oder Ethyl ersetzt sein kann,
C₍₃₋₆₎-Cycloalkyl, Phenyl,
das unsubstituiert ist oder 1, 2 oder 3 Substituenten aus der Gruppe F, Cl, Br, Methyl, Methoxy, -NR(8)R(9) trägt mit R(8) und R(9) gleich H, Methyl, Ethyl,
und worin R(5) und R(6) auch gemeinsam mit dem Stickstoffatom einen 5-, 6- oder 7-gliedrigen Ring bilden kann,
und die jeweils anderen Substituenten R(1), R(2), R(3), R(4):
Wasserstoff, F, Cl, Br, I, CF₃, NO₂, CₘF₂ₘ₊₁-CH₂-O-
mit m = 1, 2 oder 3,
R(11)-C_{q}H_{2q}-Xₚ-
mit
q = Null, 1 oder 2,
p = Null oder 1,
X = Sauerstoff,
R(11) = Wasserstoff, C₍₁₋₆₎-Alkyl, C₍₅₋₆₎-Cycloalkyl, Phenyl,
das unsubstituiert ist oder substituiert ist mit 1 oder 2 Substituenten aus der Gruppe F, Cl, Methyl und CH₃-O-,
und wobei R(1) und R(4) nicht gleichzeitig Wasserstoff ist.

3. Verbindung der Formel I nach Anspruch 1, worin bedeuten:
R(4) Wasserstoff,
einer der Substituenten R(1), R(2) oder R(3):
eine Aminogruppe 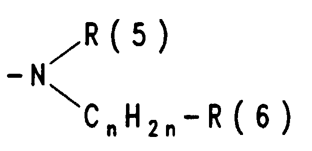 mit
R(5) = Wasserstoff oder C₍₁₋₄₎-Alkyl,
n = Null, 1 oder 2, und
R(6) = H, C₍₁₋₄₎-Alkyl C₍₅₋₆₎-Cycloalkyl, Phenyl,
das unsubstituiert ist oder 1 oder 2 Substituenten aus der Gruppe F, Cl und Methyl trägt
und die jeweils anderen Substituenten R(1), R(2), R(3) bedeuten:
Wasserstoff, F, Cl, CF₃, R(11)-C_{q}H_{2q}-Xₚ- mit
q = Null bis 2,
p = Null oder 1,
X = Sauerstoff,
R(11) = Wasserstoff, C₍₁₋₄₎-Alkyl, C₍₅₋₆₎-Cycloalkyl, Phenyl,
das unsubstituiert ist oder substituiert mit 1 oder 2 Substituenten aus der Gruppe F, Cl, Methyl und CH₃-O-,
und wobei R(1) und R(4) nicht gleichzeitig Wasserstoff bedeuten.

4. Verbindung I nach Anspruch 1, ausgewählt aus der Gruppe 2-Amino-3,5-dichlorbenzoyl-guanidin-hydrochlorid, 5-Trifluormethyl-3-N,N-dimethylaminobenzoyl-guanidin-hydrochlorid, 4-Amino-3,5-dibrombenzoyl-guanidin-hydrochlorid.

5. Verfahren zur Herstellung einer Verbindung I nach Anspruch 1, dadurch gekennzeichnet, daß man
eine Verbindung der Formel II 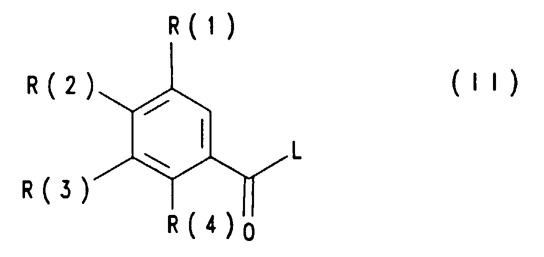 mit Guanidin umsetzt, worin R(1) bis R(4) die angegebene Bedeutung besitzen und L für eine leicht nucleophil substituierbare leaving group steht.

6. Verwendung einer Verbindung I nach Anspruch 1 zur Herstellung eines Medikaments zu Behandlung von Arrhythmien.

7. Verbindung I nach Anspruch 1 zur Verwendung in einer Methode zum Behandeln von Arrhythmien, dadurch gekennzeichnet, daß man eine wirksame Menge einer Verbindung I mit den üblichen Zusatzstoffen versetzt und in einer geeigneten Darreichungsform verabreicht.

8. Verwendung einer Verbindung I nach Anspruch 1 zur Herstellung eines Medikaments zur Behandlung oder Prophylaxe des Herzinfarktes.

9. Verwendung einer Verbindung I nach Anspruch 1 zur Herstellung eines Medikaments zur Behandlung oder Prophylaxe der Angina Pectoris.

10. Verwendung einer Verbindung I nach Anspruch 1 zur Herstellung eines Medikaments zur Behandlung oder Prophylaxe von ischämischen Zuständen des Herzens.

11. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

12. Verwendung einer Verbindung I nach Anspruch 1 zur Herstellung eines Medikaments zur Behandlung oder Prophylaxe von ischämischen Zuständen peripherer Organe und Gliedmaßen.

13. Verwendung einer Verbindung I nach Anspruch 1 zur Herstellung eines Medikaments zur Behandlung von Schockzuständen.

14. Verwendung einer Verbindung I nach Anspruch 1 zur Herstellung eines Medikaments zum Einsatz bei chirurgischen Operationen und Organtransplantationen.

15. Verwendung einer Verbindung I nach Anspruch 1 zur Herstellung eines Medikaments zur Konservierung und Lagerung von Transplantaten für chirurgische Maßnahmen.

16. Verwendung einer Verbindung I nach Anspruch I zur Herstellung eines Medikaments zur Behandlung von Krankheiten, bei denen die Zellproliferation eine primäre oder sekundäre Ursache darstellt, und somit ihre Verwendung als Antiatherosklerotika, Mittel gegen Diabetische Spätkomplikationen, Krebserkrankungen, fibrotische Erkrankungen wie Lungenfibrose, Leberfibrose oder Nierenfibrose, Prostatahyperplasie.

17. Verwendung einer Verbindung I nach Anspruch 1 zur Herstellung eines wissenschaftliches Tools zur Inhibition des Na⁺/H⁺-Exchangers, zur Diagnose der Hypertonie und proliferativer Erkrankungen.

18. Verwendung einer Verbindung I nach Anspruch I zur Herstellung eines Medikaments zur Behandlung von durch Magensäure-Hypersekretion bedingten Krankheiten.

## Claims

1. A benzoylguanidine of the formula I 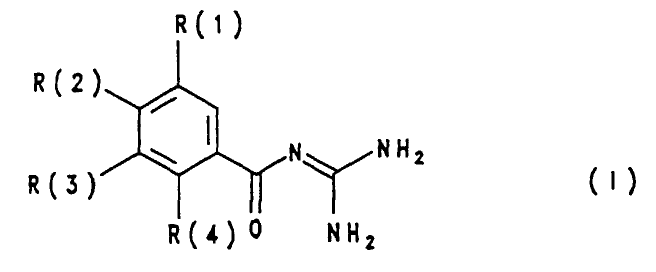 in which:
one of the substituents R(1), R(2), R(3) or R(4) is:
an amino group 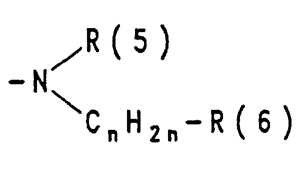 where
R(5) = hydrogen or C₍₁₋₆₎-alkyl,
n = zero, 1, 2, 3 or 4,
R(6) = H, C₍₁₋₄₎-alkyl,
in which a CH₂ group can be replaced by 1 sulfur atom or a group NR(7) where R(7) = hydrogen, methyl or ethyl,
C₍₃₋₈₎-cycloalkyl, phenyl,
which is unsubstituted or carries 1, 2 or 3 substituents from the group comprising F, Cl, Br, methyl, methoxy and -NR(8)R(9) where R(8) and R(9) are H, methyl or ethyl,
and in which
R(5) and R(6), together with the nitrogen atom, can also form a 5-, 6- or 7-membered ring in which 1 carbon atom can be replaced by oxygen, S or NR(10) where R(10) is H, C₍₁₋₃₎-alkyl or benzyl,
and the other substituents R(1), R(2), R(3) and R(4) in each case are:
hydrogen, F, Cl, Br, I, CN, CF₃, NO₂, CF₃-O-, CₘF₂ₘ₊₁-CH₂-O- where m = 1, 2 or 3,
R(11)-C_{q}H_{2q}-Xₚ-
where
q = zero, 1, 2, 3 or 4,
p = zero or 1,
X = oxygen or NR(12),
R(12) = H or C₍₁₋₃₎-alkyl,
R(11) = hydrogen, C₍₁₋₆₎-alkyl,
C₍₃₋₈₎-cycloalkyl, phenyl
which is unsubstituted or substituted by 1, 2 or 3 substituents from the group comprising F, Cl, CH₃, CH₃-O- and NR(13)R(14) where R(13) and R(14) are H, methyl or ethyl
and where R(1) and R(4) are not simultaneously hydrogen.

2. A compound of the formula I as claimed in claim 1, in which:
one of the substituents R(1), R(2), R(3) or R(4) is:
an amino group 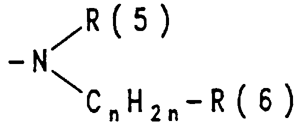 where
R(5) = hydrogen or C₍₁₋₄₎-alkyl,
n = zero, 1 or 2, and
R(6) = H, C₍₁₋₄₎-alkyl,
in which a CH₂ group can be replaced by a sulfur atom or a group NR(7) where R(7) = H, methyl or ethyl,
C₍₃₋₆₎-cycloalkyl, phenyl,
which is unsubstituted or carries 1, 2 or 3 substituents from the group comprising F, Cl, Br, methyl, methoxy and -NR(8)R(9) where R(8) and R(9) are H, methyl, ethyl,
and in which R(5) and R(6), together with the nitrogen atom; can also form a 5-, 6- or 7-membered ring,
and the other substituents R(1), R(2), R(3) and R(4) in each case are:
hydrogen, F, Cl, Br, I, CF₃, NO₂, CₘF₂ₘ₊₁-CH₂-O-
where m = 1, 2 or 3,
R(11)-C_{q}H_{2q}-Xₚ-
where
q = zero, 1 or 2,
p = zero or 1,
X = oxygen,
R(11) = hydrogen, C₍₁₋₆)-alkyl, C₍₅₋₆₎-cycloalkyl, phenyl
which is unsubstituted or is substituted by 1 or 2 substituents from the group comprising F, Cl, methyl and CH₃-O-,
and where R(1) and R(4) are not simultaneously hydrogen.

3. A compound of the formula I as claimed in claim 1, in which:
R(4) is hydrogen,
one of the substituents R(1), R(2) or R(3) is:
an amino group 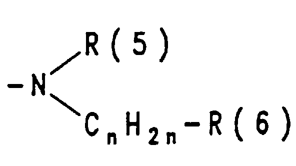 where
R(5) = hydrogen or C₍₁₋₄₎-alkyl,
n = zero, 1 or 2, and
R(6) = H, C₍₁₋₄₎-alkyl, C₍₅₋₆₎-cycloalkyl phenyl,
which is unsubstituted or carries 1 or 2 substituents from the group comprising F, Cl and methyl
and the other substituents R(1), R(2) and R(3) in each case are:
hydrogen, F, Cl, CF₃, R(11)-C_{q}-H_{2q}-Xₚ- where
q = zero to 2,
p = zero or 1,
X = oxygen,
R(11) = hydrogen, C₍₁₋₄₎-alkyl, C₍₅₋₆₎-cycloalkyl, phenyl
which is unsubstituted or substituted by 1 or 2 substituents from the group comprising F, Cl, methyl and CH₃-O-,
and where R(1) and R(4) are not simultaneusly hydrogen.

4. A compound I as claimed in claim 1, selected from the group comprising 2-amino -3,5-dichlorobenzoylguanidine hydrochloride, 5-trifluoromethyl-3-N,N-dimethylaminobenzoylguanidine hydrochloride, 4-amino-3,5-dibromobenzoylguanidine hydrochloride.

5. A process for the preparation of a compound I as claimed in claim 1, which comprises reacting
a compound of the formula II 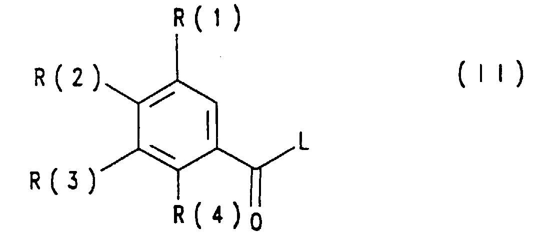 in which R(1) to R(4) have the given meaning and L is a leaving group which can be easily nucleophilically substituted, with guanidine.

6. The use of a compound I as claimed in claim 1 for the production of a medicament for the treatment of arrhythmias.

7. A compound I as claimed in claim 1 for use in a method for treating arrhythmias, wherein an effective amount of a compound I is mixed with the customary additives and brought into a suitable administration form.

8. The use of a compound I as claimed in claim 1 for the production of a medicament for the treatment or prophylaxis of cardiac infarct.

9. The use of a compound I as claimed in claim 1 for the production of a medicament for the treatment or prophylaxis of angina pectoris.

10. The use of a compound I as claimed in claim 1 for the production of a medicament for the treatment or prophylaxis of ischemic conditions of the heart.

11. The use of a compound I as claimed in claim 1 for the production of a medicament for the treatment or prophylaxis of ischemic conditions of the peripheral and central nervous system and of a stroke.

12. The use of a compound I as claimed in claim 1 for the production of a medicament for the treatment or prophylaxis of ischemic conditions of peripheral organs and limbs.

13. The use of a compound I as claimed in claim 1 for the production of a medicament for the treatment of states of shock.

14. The use of a compound I as claimed in claim 1 for the production of a medicament for use in surgical operations and organ transplantation.

15. The use of a compound I as claimed in claim 1 for the production of a medicament for the preservation and storage of transplants for surgical measures.

16. The use of a compound I as claimed in claim 1 for the production of a medicament for the treatment of diseases in which cell proliferation is a primary or secondary cause, and thus their use as antiatherosclerotics, agents against diabetic late complications, cancers, fibrotic diseases such as pulmonary fibrosis, fibrosis of the liver or fibrosis of the kidneys and prostate hyperplasia.

17. The use of a compound I as claimed in claim 1 for the production of a scientific tool for the inhibition of the Na⁺/H⁺ exchanger, for the diagnosis of hypertension and proliferative disorders.

18. The use of a compound I as claimed in claim 1 for the production of a medicament for the treatment of diseases caused by gastric acid hypersecretion.

## Revendications

1. Benzoylguanidines de formule I 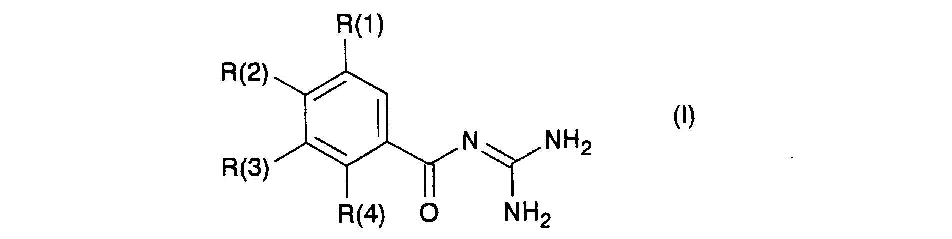 dans laquelle :
un des substituants R(1), R(2), R(3) ou R(4) représente :
un groupe amino 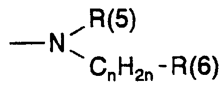 dans lequel
R(5) = un atome d'hydrogène ou un groupe alkyle C₍₁₋₆₎,
n = 0, 1, 2, 3 ou 4,
R(6) = H, un groupe alkyle C₍₁₋₄₎,
dans lequel un groupe CH₂ peut être remplacé par un atome de S ou un groupe NR(7) avec R(7) = un atome d'hydrogène, un groupe méthyle ou éthyle
un groupe cycloalkyle C(₃₋₈), phényle
celui-ci étant non substitué ou substitué par 1, 2 ou 3 substituants choisis parmi F, CI, Br et les groupes méthyle, méthoxy et -NR(8)R(9), R(8) et R(9) étant un H, un groupe méthyle, éthyle
et où
R(5) et R(6) peuvent représenter ensemble avec l'atome d'azote un cycle à 5, 6 ou 7 chainons dans lequel un atome de carbone peut être remplacé par un atome d'oxygène, de S ou par un groupe NR(10), avec R(10) = H, un groupe alkyle C₍₁₋₃₎ ou benzyle,
et les autres substituants R(1), R(2), R(3), R(4) sont :
un atome d'hydrogène, F, CI, Br, I, un groupe CN, CF₃, NO₂, CF₃-O-, CₘF₂ₘ₊₁-CH₂-O
avec m = 1, 2 ou 3,
R(11)-C_{q}H_{2q}-Xₚ-
avec
q = 0, 1, 2, 3 ou 4,
p = 0 ou 1
X = un atome d'oxygène ou un groupe NR(12),
R(12) = H ou un groupe alkyle C₍₁₋₃₎
R(11)= un atome d'hydrogène, un groupe alkyle C₍₁₋₆₎, cycloalkyle C₍₃₋₈₎, phényle,
celui-ci étant non substitué ou substitué par 1, 2 ou 3 substituants choisis parmi F, CI et les groupes CH₃, CH₃-O-, -NR(13)R(14), R(13) et R(14) étant H, un groupe méthyle ou éthyle,
et où R(1) et R(4) ne sont pas simultanément un atome d'hydrogène.

2. Composé de formule I selon la revendication 1, où les groupes ont les significations suivantes :
un des substituants R(1), R(2), R(3) ou R(4) est:
un groupe amino 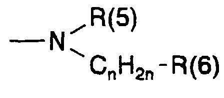 dans lequel
R(5) = un atome d'hydrogène ou un groupe alkyle C₍₁₋₄₎,
n = 0, ou 2 et
R(6) = H, un groupe alkyle C₍₁₋₄₎,
dans lequel un groupe CH₂ peut être remplacé par un atome de S ou un groupe NR(7) avec R(7) = H, un groupe méthyle ou éthyle
un groupe cycloalkyle C₍₃₋₆₎, phényle
celui-ci étant non substitué ou substitué par 1, 2 ou 3 substituants choisis parmi F, CI, Br et les groupes méthyle, méthoxy et -NR(8)R(9), R(8) et R(9) étant un H, un groupe méthyle, éthyle
et où R(5) et R(6) peuvent représenter ensemble avec l'atome d'azote un cycle à 5, 6 ou 7 chainons,
et les autres substituants R(1), R(2), R(3), R(4) sont:
un atome d'hydrogène, F, CI, Br, I, un groupe CF₃, NO₂, CₘF₂ₘ₊₁-CH₂-O
avec m = 1, 2 ou 3,
R(11)-C_{q}H_{2q}-Xₚ-
avec
q = 0, 1 ou 2,
p = 0 ou 1
X = un atome d'oxygène
R(11) = un atome d'hydrogène, un groupe alkyle C₍₁₋₆₎, cycloalkyle C₍₅₋₆₎, phényle,
celui-ci étant non substitué ou substitué par 1 ou 2 substituants choisis parmi F, CI et les groupes méthyle et CH₃-O-
et où R(1) et R(4) ne sont pas simultanément un atome d'hydrogène.

3. Composé de formule I selon la revendication 1, où les groupes ont les significations suivantes :
R(4) est un atome d'hydrogène
un des substituants R(1), R(2) ou R(3) est :
un groupe amino 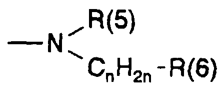 dans lequel
R(5) = un atome d'hydrogène ou un groupe alkyle C₍₁₋₄₎,
n = 0, 1 ou 2 et
R(6) = H, un groupe alkyle C₍₁₋₄₎, cycloalkyle C₍₅₋₆₎, phényle
celui-ci étant non substitué ou substitué par 1 ou 2 substituants choisis parmi F, CI et le groupe méthyle,
et les autres substituants R(1), R(2), R(3) sont :
un atome d'hydrogène, F, CI, un groupe CF₃, R(11)-C_{q}H_{2q}-Xₚ- avec
q = 0 à 2,
p = 0 ou 1,
X = un atome d'oxygène,
R(11) = un atome d'hydrogène, un groupe alkyle C₍₁₋₄₎, cycloalkyle C₍₅₋₆₎, phényle,
celui-ci étant non substitué ou substitué par 1 ou 2 substituants choisis parmi F, CI et les groupes méthyle et CH₃-O-,
et où R(1) et R(4) ne sont pas simultanément un atome d'hydrogène.

4. Composé I selon la revendication 1, choisi parmi le chlorhydrate de 2-amino-3,5-dichlorobenzoyl-guanidine, le chlorhydrate de 5-trifluorométhyl-3-N,N-diméthylaminobenzoyl-guanidine, le chlorhydrate de 4-amino-3,5-dibromobenzoyl-guanidine.

5. Procédé pour la préparation d'un composé I selon la revendication 1, caractérisé en ce que l'on fait réagir un composé de formule Il 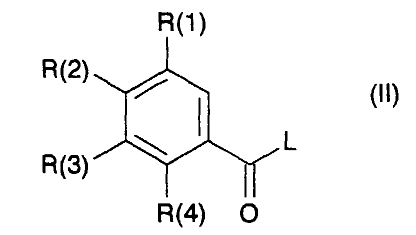 avec de la guanidine, les groupes R(1) à R(4) ayant les significations données et L signifiant un groupe partant, facilement substituable par un nucléophile.

6. Utilisation d'un composé I selon la revendication 1 pour la préparation d'un médicament pour le traitement d'arythmies.

7. Composé I selon la revendication 1 pour l'utilisation dans une méthode pour le traitement d'arythmies, caractérisée en ce que l'on mélange une quantité active d'un composé I avec les adjuvants courants et qu'on les met sous une forme de présentation appropriée.

8. Utilisation d'un composé I selon la revendication 1 pour la préparation d'un médicament pour le traitement ou la prophylaxie de l'infarctus du myocarde.

9. Utilisation d'un composé I selon la revendication 1 pour la préparation d'un médicament pour le traitement ou la prophylaxie de l'angine de poitrine.

10. Utilisation d'un composé I selon la revendication 1 pour la préparation d'un médicament pour le traitement ou la prophylaxie des états ischémiques du coeur.

11. Utilisation d'un composé I selon la revendication 1 pour la préparation d'un médicament pour le traitement ou la prophylaxie des états ischémiques du système nerveux périphérique et central et de l'apoplexie.

12. Utilisation d'un composé I selon la revendication 1 pour la préparation d'un médicament pour le traitement ou la prophylaxie des états ischémiques des organes périphériques et des membres.

13. Utilisation d'un composé I selon la revendication 1 pour la préparation d'un médicament pour le traitement des états de choc.

14. Utilisation d'un composé I selon la revendication 1 pour la préparation d'un médicament à utiliser au cours d'opérations chirurgicales et de transplantations d'organes.

15. Utilisation d'un composé I selon la revendication 1 pour la préparation d'un médicament pour la conservation et le stockage de transplants à des fins chirurgicales.

16. Utilisation d'un composé I selon la revendication 1 pour la préparation d'un médicament pour le traitement de maladies dont une cause primaire ou secondaire est représentée par la prolifération cellulaire, et ainsi son utilisation comme antiathérosclérotique, comme agent contre les complications tardives du diabète, les maladies cancéreuses, les maladies fibrotiques telles que les fibroses des poumons, du foie ou des reins, l'hyperplasie de la prostate.

17. Utilisation d'un composé I selon la revendication 1 pour la préparation d'un outil scientifique pour l'inhibition de l'échangeur-Na⁺/H⁺ pour le diagnostic de l'hypertonie et de maladies prolifératives.

18. Utilisation d'un composé I selon la revendication 1 pour la préparation d'un médicament pour le traitement de maladies provoquées par l'hypersécrétion d'acides gastriques.
